# EUROPÄISCHE PATENTSCHRIFT

(11) **EP 0 357 999 B1**
(45) Veröffentlichungstag und Bekanntmachung des Hinweises auf die Patenterteilung: **16.03.1994**
(21) Anmeldenummer: 89114963.5
(22) Anmeldetag: 12.08.1989
(51) Int. Cl.: A61B 17/34, A61M 19/00, A61M 25/06, A61M 5/00

(54) **Besteck für die intrapleurale Regionalanästhesie**
Set of instruments for intrapleural local anaesthesia
Jeu d'instruments pour anesthésie localee intrapleurale

(30) Priorität: 09.09.1988 DE 3830653
(43) Veröffentlichungstag der Anmeldung: 14.03.1990
(73) Patentinhaber: B. Braun Melsungen AG, 34209 Melsungen (DE)
(72) Erfinder: Sydow, Friedrich-Wilhelm, Dr. Städt. Kliniken Kas., D-3500 Kassel (DE); Haindl, Hans-Günter, Dr., D-4508 Melsungen (DE)
(74) Vertreter: Selting, Günther, Dipl.-Ing.

(56) Entgegenhaltungen:
- EP-A- 0 091 846
- EP-A- 0 185 864
- WO-A-88/03420
- DE-A- 1 956 206
- US-A- 2 001 638

## Beschreibung

Die Erfindung bezieht sich auf ein Besteck für die intrapleurale Regionalanästhesie, bestehend aus einer Kanüle, die an einem Ende einen Kanülenansatz und am anderen Ende eine angeschliffene Zentralöffnung aufweist,
aus einem das Lumen der Kanüle ausfüllenden Mandrin und aus einem in der Kanüle längsverschiebbaren Katheter.

Bei der Regionalanästhesie werden Narkotika in die Nähe von Nerven injiziert. Eine Form dieser Anästhesie ist die Epiduralanästhesie mit Punktion des Epiduralraumes im thorakalen oder lumbalen Gebiet und Injektion eines Lokalanästhetikums. Dabei wird die Kanüle durch das Ligamentum supra- und interspinale gestochen und gelangt nach Überwindung des derben Ligamentum flavum und des Periostes in den Epiduralraum. Eine als Tuohy-Kanüle bezeichnete Kanüle mit seitwärts gerichteter angeschliffener Zentralöffnung erlaubt die gefahrlose Punktion des Epiduralraumes mit minimaler Verletzungsgefahr der Dura. Das Erreichen des Epiduralraumes erkennt der Anwender an dem Nachlassen des Injektionswiderstandes einer auf die Kanüle aufgesetzten Spritze (loss of resistance). Das Anästhetikum kann durch die Kanüle injiziert oder durch einen in den Epiduralraum eingeführten Katheter appliziert werden.

Aufgrund einer mißglückten Katheter-Epiduralanästhesie, bei der der Katheter versehentlich in den Pleuraspalt geschoben wurde, haben norwegische Mediziner entdeckt, daß die Verabreichung von Lokalanästhetika in den Pleuraspalt für bestimmte Operationen zu einer ausgezeichneten Analgesie (Aufhebung der Schmerzempfindung) führt. Insbesondere bei Gallen- und Magenoperationen ist mit diesem Verfahren der intrapleuralen Regionalanästhesie eine gute Analgesie zu erzielen; dabei ist das Verfahren einfacher und risikoärmer als die thorakale Epiduralanästhesie.

Bei der pleuralen Katheteranalgesie wird bisher mit herkömmlichen Epiduralkatheter-Bestecken gearbeitet, die aus einer Tuohy-Kanüle, einem das Lumen der Kanüle ausfüllenden Mandrin und einem in der Kanüle längsverschiebbaren Katheter bestehen. Bei der Verwendung solcher Epiduralkatheter-Bestecke für die intrapleurale Regionalanästhesie ergeben sich einige Probleme:
während bei der Epiduralanästhesie die Kanüle durch das derbe Ligamentum interspinale sticht, wird sie bei der intrapleuralen Regionalanästhesie durch subkutanes Fettgewebe geschoben, das sehr locker ist, so daß die loss of resistance-Technik zur Lokalisation des Pleuraspaltes nicht zufriedenstellend funktioniert. Mit einer an den Kanülenansatz angeschlossenen Spritze läßt sich Kochsalzlösung relativ frei in das lockere Gewebe injizieren und ein eindeutiger Abfall des Injektionswiderstandes im Pleuraspalt ist nicht festzustellen. Als Ausweg wird hier teilweise mit der Infusion über die Tuohy-Kanüle gearbeitet, an deren Kanülenansatz ein Schlauch angeschlossen wird, der mit einer Infusionsflasche in Verbindung steht. Dies ist sehr umständlich und auch nicht 100%ig zuverlässig. Bei Erreichen des Pleuraspaltes wird nach dem Abziehen der Spritze oder der Infusion von der Tuohy-Kanüle und vor dem Einfädeln und Vorschieben des Katheters durch die Tuohy-Kanüle Luft in den Pleuraspalt gesogen. Hierdurch kommte es zu einem Pneumothorax. Ein kleiner Pneumothorax ist erwünscht, um den Raum für das Vorschieben des Katheters freizugeben und einer Perforation der Lunge vorzubeugen. Wird der Pneumothorax hingegen zu groß, läuft er dem eigentlichen Ziel der Analgesie, nämlich der Verbesserung der Atemfunktion, entgegen. Außerdem ist nachteilig, daß die Längen der Standard-Epiduralkanülen bekannter Bestecke häufig für adipöse Patienten nicht ausreichen, bei denen die Indikation zur postoperativen Analgesie besonders dringlich ist.

Der Erfindung liegt die Aufgabe zugrunde, die Durchführung der intrapleuralen Regionalanästhesie einfacher und durch verbesserte Lokalisierung des Pleuraspaltes sicherer zu machen.

Diese Aufgabe wird erfindungsgemäß dadurch gelöst, daß mit dem Kanülenansatz ein Y-Rohrstück lösbar verbunden ist, das am Ende seines zu der Kanüle koaxialen Rohrteiles mit einer abdichtenden Katheterschleuse ausgestattet ist und dessen abzweigender Rohrteil offen mit einem unverformten kollabierfähigen Beutelkörper aus Elastomermaterial in Verbindung steht.

Bei einem derartigen Besteck dient der unverformte hochflexible Beutelkörper aus Elastomermaterial am Ende des abzweigenden Rohrteiles als Detektor zur Anzeige des Erreichens des Pleuraspaltes durch Zusammenziehung unter dem Einfluß des in dem Pleuraspalt herrschenden Unterdruckes, wobei die abdichtende Katheterschleuse an dem zu der Kanüle koaxialen Rohrteil das Einschleppen von Außenluft in das Kanalsystem des Bestecks sowohl bei eingesetztem Katheter als auch ohne Katheter verhindert. Auf diese Weise wird erreicht, daß das Besteck die Durchführung der pleuralen Analgesie für den Anwender einfacher und für den Patienten sicherer macht.

In vorteilhafter Ausgestaltung der Erfindung ist vorgesehen, daß die Katheterschleuse einen Gummistopfen mit einem radial zusammenquetschbaren zentralen Durchlaß für den Katheter aufweist. Die radiale Zusammenquetschbarkeit des zentralen Durchlasses erlaubt seine Einengung derart, daß der Katheter verschiebbar und dabei auf seinem Außenumfang zuverlässig abgedichtet ist. Bei weiterer Verengung des Durchlasses des Gummistopfens wird der Katheter in dem Durchlaß blockiert, so daß er unverschieblich in dem zu der Kanüle koaxialen Rohrteil steckt. Der Katheter wird fabrikseitig so in dem Y-Rohrstück festgelegt, daß seine Spitze vor der Mündungsöffnung des abzweigenden Rohrteiles liegt und der Unterdruck im Pleuraspalt ungehindert auf die Innenfläche des hochflexiblen Beutelkörpers zur Einwirkung kommt. Eine an den Katheter abnehmbar angesetzte Klemme verhindert das Eindringen von Luft durch das Katheterlumen. Da vorzugsweise ein weicher Epiduralkatheter verwendet wird, genügt eine Nabelklemme zum Verschluß des Katheters.

Der kollabierfähige Beutelkörper kann als Ballon ausgebildet sein. Bevorzugt besteht er aus einem Gummifingerling, der in einer mit dem abzweigenden Rohrteil verbundenen steifen durchsichtigen Schutzkappe untergebracht ist. Der Gummifingerling und die Schutzkappe haben vorzugsweise eine zylindrische Wandung und sind am Ende durch einen kuppelförmigen Boden verschlossen. Diese Formgebung begünstigt eine ungestörte Handhabung des Bestecks und erlaubt eine raumsparende Verpackung. Der in der steifen Schutzkappe geschützte Gummifingerling ist so klein, daß nur eine sehr geringe unschädliche Luftmenge in den Pleuraspaltraum eingesogen wird.

Die Kanüle und der Mandrin haben vorzugsweise eine Länge von 100 mm bis 150 mm. Eine solche im Vergleich zu einem bekannten Epiduralkatheterbesteck überlange Kanülen-/Mandrinausbildung ermöglicht die problemlose Durchführung der pleuralen Analgesie auch bei adipösen Patienten.

Nach Lokalanästhesie wird die Tuohy-Kanüle mit Mandrin vorgeschoben, bis sie etwa zwischen den Rippen liegt. Dann wird der Mandrin axial aus der Kanüle herausgezogen und es wird das Y-Rohrstück mit eingesetztem Katheter und nach unten hängendem Beutelkörper auf den Kanülenansatz aufgesteckt. Nun wird die Kanüle langsam weitergeschoben. Sobald die Zentralöffnung der Kanüle den Pleuraspalt erreicht hat, kollabiert der hochflexible unverformte Beutelkörper durch den im Pleuraspalt herrschenden Unterdruck. Der Anwender erkennt durch diesen Indikator sofort den Eintritt der Kanülenspitze in den Pleuraspalt und weiß, daß dort ein vom Volumen her definierter kleiner Pneumothorax gesetzt wird.

Die Kanüle wird jetzt noch ca. 3 mm weiter vorgeschoben; danach wird die Katheterschleuse so weit gelockert, daß der Katheter sich vorschieben läßt, ohne daß jedoch Luft in das Y-Rohrstück eintritt. Da der Katheter an seinem äußeren Ende durch die Klemme verschlossen ist, kann auch durch sein Lumen keine Luft in das System eindringen.

Wenn die Spitze des Katheters in der korrekten Position liegt, wird die Kanüle mit dem Y-Rohrstück über den Katheter zurückgezogen und der Katheter wird vor der Kanüle abgeklemmt, um die Kanüle und das Y-Rohrstück vollkommen von dem Katheter abziehen zu können. Wegen des geringen Innenlumens des Katheters kann das Abklemmen vor der Kanüle mit der gleichen Klemme wie hinter dem Y-Rohrstück erfolgen, da in der Zeit des Umsetzens der Klemme kaum nennenswerte Luftmengen in den Katheter eintreten können.

Nach Abziehen der Kanüle und des Y-Rohrstückes von dem Katheter wird an sein äußeres Ende eine übliche Katheterkupplung angeschraubt, auf die ein Bakterienfilter aufgesetzt und mit einem Stopfen verschlossen wird. Jetzt kann die Klemme von dem Katheter entfernt werden und das Besteck zur intrapleuralen Regionalanästhesie ist injektionsbereit.

In der Zeichnung ist ein Ausführungsbeispiel der Erfindung schematisch dargestellt.
Fig. 1 zeigt ein Besteck zur intrapleuralen Regionalanästhesie vor der Anwendung, teilweise längsgeschnitten und
Fig. 2 das Besteck nach Figur 1 bei der Anwendung.

Das Besteck zur pleuralen Katheteranalgesie mit einer Detektoreinrichtung zur Anzeige des Erreichens des Pleuraspaltes besteht im wesentlichen aus einer Kanüle 10 aus Stahl, die an einem Kanülenansatz 11 befestigt ist und aus einem Y-Rohrstück 20 sowie einem durch den geraden Durchlaß von Kanüle 10 und Y-Rohrstück 20 hindurchschiebbaren knickunanfälligen Katheter 30 aus Polyamid, der Markierungsringe für die exakte Kontrolle der Einführungstiefe aufweist und in der Nähe des Y-Rohrstückes 20 mit einer lösbaren Klemme 31, z.B. einer üblichen Nabelklemme, abgeklemmt ist.

Bei der Kanüle 10 handelt es sich um eine sogenannte "Tuohy-Kanüle", deren Zentralöffnung 12 seitwärts gerichtet und mit einem Spezialschliff versehen ist. Zur Anwendung bei adipösen Patienten hat die Kanüle 10 Überlängen zwischen 100 mm bis 150 mm. In der Kanüle 10 steckt ein ihr Lumen ausfüllender Mandrin, der nach der Punktion mittels des Kopfstückes 13 aus der Kanüle 10 herausgezogen wird.

Das Y-Rohrstück 20 kann aus transparentem Kunststoff gefertigt sein. Es weist einen zu der Kanüle 10 koaxialen geraden Rohrteil 21 mit einem konzentrischen geraden Durchlaß 22 auf, der an beiden Enden offen ist. An dem kanülenseitigen Ende des Rohrteiles 21 befindet sich ein Außenkonus 23, der in einen Innenkonus des Kanülenansatzes 11 lösbar, aber klemmend einsteckbar ist. Am gegenüberliegenden Ende des Rohrteiles 21 ist eine zylindrische Kammer zur Aufnahme eines in diese passenden Gummistopfens 40 ausgebildet. Die Kammer und der Gummistopfen 40 sind am inneren Ende konisch verjüngt ausgebildet. Eine Schraubkappe 24 mit einem Zentralansatz 25 ist auf ein Außengewinde 26 des äußeren Endes des Rohrteiles 21 aufschraubbar. Dabei preßt die gerade Stirnfläche des Zentralansatzes 25 gegen die ebenfalls gerade äußere Stirnfläche des Stopfens 40. Sowohl der Stopfen 40 als auch die Kappe 24 sind mit zentralen Durchlässen für den Katheter 30 versehen. Durch Anziehen bzw. Lockern der Schraubkappe 24 wird der Durchlaß des Stopfens 40 radial verengt oder erweitert, damit der Katheter 30 auf seinem Außenumfang abgedichtet und dabei axial verschiebbar oder blockiert ist.

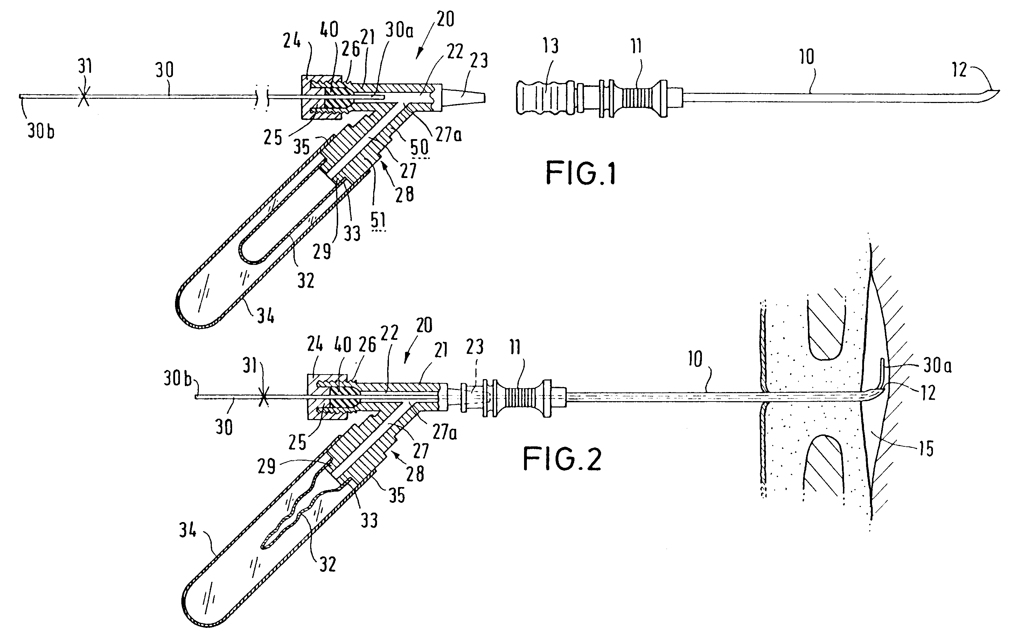

In dem in Figur 1 gezeigten Anlieferzustand des Bestecks ist der Katheter 30 in dem Y-Rohrstück 20 so festgelegt, daß seine Spitze 30a vor der Mündung 27a eines von dem Durchlaß 22 schräg abzweigenden Kanales 27 liegt. Der Kanal 27 durchsetzt zentral einen von dem zu der Kanüle 10 koaxialen Rohrteil 21 abzweigenden Rohrteil 28. Der abzweigende Rohrteil 28 kann mit dem Rohrteil 21 insgesamt einstückig geformt sein oder er kann aus einem angeformten kurzen Stutzen 50 und einem über eine Luer-Lock-Verbindung lösbar angekuppelten Anschlußstück 51 gebildet sein. Das äußere Ende des abzweigenden Rohrteiles 28 ist mit einem Fortsatz 29 geringeren Durchmessers versehen, auf den ein länglicher zylindrischer Gummifingerling 32 mit seinem verstärkten Rand 33 aufgespannt ist. Der Gummifingerling 32 ist sehr dünnwandig, so daß er hochflexibel ist und sich bei geringsten Unter- oder Überdrücken deformiert. Im Grundzustand ist der Gummifingerling 32 unverformt, d.h. er hat gemäß dem gezeichneten Beispiel die Form eines sehr kleinen Reagenzglases mit zylindrischer Wand und rundem Boden. Der Gummifingerling 32 ist konzentrisch von einer Schutzkappe 34 umgeben, die aus durchsichtigem Kunststoffmaterial besteht und steif ist. Die Form der Schutzkappe 34 entspricht im wesentlichen der Form des Gummifingerlings 32, jedoch hat sie einen größeren Durchmesser und größere Länge als dieser. Der kreisförmige Rand 35 der Schutzkappe 34 ist auf den kreisförmigen Außenumfang des Rohrteiles 28 festsitzend aufgeschoben. Durch die Schutzkappe 34 läßt sich der Zustand des Gummifingerlings 32 beobachten. Verformungen des Gummifingerlings 32 während der Anwendung werden durch die Schutzkappe 34 nicht behindert. Ihre Aufgabe ist es, den Gummifingerling 32 gegen Berührung durch den Anwender zu schützen, um diesem die Handhabung des Bestecks zu erleichtern.

Zur Durchführung der pleuralen Katheteranalgesie wird nach Lokalanästhesie die Kanüle 10 mit Mandrin vorgeschoben, bis sie etwa zwischen den Rippen liegt. Nach Herausziehen des Mandrins wird das Y-Rohrstück 20 mittels des Außenkonus 23 mit dem Kanülenansatz 11 zusammengesteckt (Fig. 2). Wenn bei langsamem Weiterschieben der Kanüle 10 der Pleuraspalt 15 erreicht ist, kollabiert der Gummifingerling 32 durch den im Pleuraspalt 15 herrschenden Unterdruck und der Anwender wird über den Eintritt der Kanülenöffnung 12 in den Pleuraspalt 15 informiert. Sodann wird nach geringfügigem weiterem Vorschub der Kanüle 10 die Schraubkappe 24 etwas herausgedreht, so daß der Durchlaß des Gummistopfens 40 so weit vergrößert wird, daß der Katheter 30 sich vorschieben läßt, jedoch keine Luft an ihm vorbei in das System eindringen kann. Die Klemme 31 verhindert ein Einschleppen von Luft durch das Lumen des Katheters 30. Wenn die Spitze 30a des Katheters 30 die dargestellte korrekte Position in dem Pleuraspalt 15 eingenommen hat, wird die Kanüle 10 mit dem Y-Rohrstück 20 nach hinten von dem Katheter 30 abgezogen, nachdem die Klemme 31 abgenommen worden und vor der Kanüle 10 wieder auf den Katheter 30 aufgeklemmt worden ist. Sodann wird auf das freie Ende 30b des Katheters wie übliche eine Katheterkupplung aufgeschraubt, ein Filter aufgesetzt und ein Stopfenverschluß angebracht. Nach Entfernen der versetzten Klemme 31 ist das System injektionsbereit.

## Patentansprüche

1. Besteck für die intrapleurale Regionalanästhesie, bestehend aus einer Kanüle (10), die an einem Ende einen Kanülenansatz (11) und am anderen Ende eine angeschliffene Zentralöffnung (12) aufweist,
aus einem das Lumen der Kanüle (10) ausfüllenden Mandrin
und aus einem in der Kanüle (10) längsverschiebbaren Katheter (30),
**dadurch gekennzeichnet**, daß mit dem Kanülenansatz (11) ein Y-Rohrstück (20) lösbar verbunden ist, das am Ende seines zu der Kanüle (10) koaxialen Rohrteiles (21) mit einer abdichtenden Katheterschleuse (24, 25,40) ausgestattet ist und dessen abzweigender Rohrteil (28) offen mit einem unverformten kollabierfähigen Beutelkörper (32) aus Elastomermaterial in Verbindung steht.

2. Besteck nach Anspruch 1,
**dadurch gekennzeichnet**, daß die Katheterschleuse einen Gummistopfen (40) mit einem radial zusammenquetschbaren zentralen Durchlaß für den Katheter (30) aufweist.

3. Besteck nach Anspruch 1 oder 2,
**dadurch gekennzeichnet,** daß der Beutelkörper als hochflexibler Gummifingerling (32) ausgebildet ist, der in einer mit dem abzweigenden Rohrteil (28) verbundenen steifen durchsichtigen Schutzkappe (34) untergebracht ist.

4. Besteck nach einem der Ansprüche 1 bis 3,
**dadurch gekennzeichnet**, daß an den Katheter (30) eine Klemme (31) abnehmbar angesetzt ist.

5. Besteck nach einem der Ansprüche 1 bis 4,
**dadurch gekennzeichnet**, daß die Kanüle (10) und der Mandrin 130 mm bis 150 mm lang sind.

## Claims

1. Intrapleural regional anaesthesia set comprising a needle (10) having a needle hub (11) at one end and a ground central opening (12) at the other end,
a stylet filling the lumen of the needle (10)
and a catheter (30) being longitudinally displaceable in the needle (10),
**characterised in that**
a Y-pipe member (20) is detachably connected with the needle hub (11), being provided with a sealing catheter introducing sluice (24, 25, 40) at the end of the pipe portion (21) coaxial with the needle (10), and the branching pipe portion (28) of which is in open communication with a non-deformed collapsible bag member (32) of elastomeric material.

2. The set according to claim 1, characterised in that the catheter introducing sluice is provided with a rubber stopper (40) with a radially squeezable central passage for the catheter (30).

3. The set according to claim 1 or 2, characterised in that the bag member is provided as a highly flexible finger-stall (32) disposed in a rigid transparent protective cap (34) connected with the branching pipe portion (28).

4. The set according to one of claims 1 to 3, characterised in that a clamp (31) is removably disposed at the catheter (30).

5. The set according to one of claims 1 to 4, characterised in that the needle (10) and the stylet are 130 to 150 mm in length.

## Revendications

1. Nécessaire pour l'anesthésie locale intrapleurale, constitué par une canule (10), qui possède, à une extrémité, un embout de canule (11) et à l'autre extrémité une ouverture centrale affûtée (12),
par un mandrin qui remplit la lumière de la canule (10), et par un cathéter (30) apte à être déplacé longitudinalement dans la canule (10),
caractérisé en ce qu'à l'embout (11) de la canule est raccordé de façon amovible un élément tubulaire en Y (20), qui comporte, à l'extrémité de sa partie tubulaire (21) coaxiale à la canule (10), un sas de cathéter (24,25,40), réalisant une fermeture étanche et dont la partie tubulaire ouverte (28), placée en dérivation, est reliée à un corps formant sachet non déformé (32), apte à être aplati et formé d'un matériau élastomère.

2. Nécessaire selon la revendication 1, caractérisé en ce que le sas du cathéter comporte un bouchon de caoutchouc (40) pourvu d'un passage central, qui peut être fermé par compression radiale, pour le cathéter (30).

3. Nécessaire selon la revendication 1 ou 2, caractérisé en ce que le corps formant sachet est réalisé sous la forme d'un doigt en caoutchouc très flexible (32), qui est logé dans un capot de protection rigide et transparent (34), qui est relié à l'élément tubulaire (28) disposé en dérivation.

4. Nécessaire selon l'une des revendications 1 à 3, caractérisé en ce qu'une pince (31) peut être montée de façon amovible sur le cathéter (30).

5. Nécessaire selon l'une des revendications 1 à 4, caractérisé en ce que la canule (10) et le mandrin possèdent une longueur comprise entre 130 mm et 150 mm.
